(19) Europäisches Patentamt
European Patent Office
Office européen des brevets

(11) Veröffentlichungsnummer: **0 184 061**
**B1**

(12) # EUROPÄISCHE PATENTSCHRIFT

(45) Veröffentlichungstag der Patentschrift: **14.12.88**

(51) Int. Cl.⁴: **C 07 C 91/06**, A 61 K 7/13

(21) Anmeldenummer: **85114608.4**

(22) Anmeldetag: **16.11.85**

(54) **4-(N-Ethyl,N-2'-hydroxyethyl)-amino-1-(2"-hydroxyethyl)-amino-2-nitro-benzol und Mittel zur Färbung von Haaren.**

(30) Priorität: **24.11.84 DE 3442861**

(43) Veröffentlichungstag der Anmeldung: **11.06.86 Patentblatt 86/24**

(45) Bekanntmachung des Hinweises auf die Patenterteilung: **14.12.88 Patentblatt 88/50**

(84) Benannte Vertragsstaaten: **AT BE CH DE FR GB IT LI LU NL SE**

(56) Entgegenhaltungen:
**FR-A-1 454 314**
**FR-E-85 850**

**Merck-Index, 10th ed., Seiten 550 und 644 (1983)**
**Int. J. Cosm. Sci. 4, 25-35 (1982)**
**Mutation Research 31, 347-364 (1975)**

**Die Akte enthält technische Angaben, die nach dem Eingang der Anmeldung eingereicht wurden und die nicht in dieser Patentschrift enthalten sind.**

(73) Patentinhaber: **Wella Aktiengesellschaft, Berliner Allee 65, D-6100 Darmstadt (DE)**

(72) Erfinder: **Clausen, Thomas, Dr., Ernst Pasqué Strasse 35A, D-6146 Alsbach (DE)**
Erfinder: **Konrad, Eugen, Mecklenburger Strasse 101, D-6100 Darmstadt (DE)**

**EP 0 184 061 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft die neue Verbindung der Formel I und mit Hilfe von I hergestellte Mittel zum Farben von Haaren.

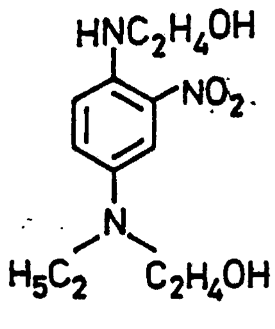

(I)

Nitrofarbstoffe finden heute in Haarfärbemitteln eine breite Anwendung. Durch die Kombination mehrerer verschieden gefärbter Nitrofarbstoffe lassen sich Haare ohne die Anwendung von Oxidationsmitteln in natürlichen bis modischen Nuancen einfärben.

Dabei lassen sich natürliche Haarfarben herstellen durch die Kombination eines blauen mit einem orangen Farbstoff oder durch die Kombination eines blauen mit einem rein gelben und einem roten Nitrofarbstoff.

Es besteht daher in jedem Falle ein Bedarf nach einem blauen Nitrofarbstoff, an den aber noch viele weitere Anforderungen gestellt werden. So muß er in toxikologischer und dermatologischer Hinsicht unbedenklich sein und die Erzielung von Färbungen in der gewünschten Intensität ermöglichen, was unter anderem auch eine ausreichende Wasserlöslichkeit voraussetzt. Außerdem wird für die erzielten Haarfärbungen eine gute Licht-, Säure- und Reibechtheit gefordert.

Zur Erzielung blauer Nitrofarbstoffe werden in der Regel trialkylierte 2-Nitro-p-phenylen-diaminderivate der allgemeinen Formel II hergestellt.

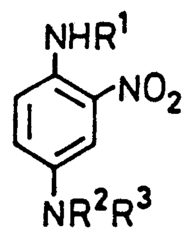

(II)

Es hat bereits zahlreiche Versuche gegeben, Farbstoffe darzustellen, die den obigen Anforderungen entsprechen. So wird ein Isomeres der Verbindung I, die 1-Ethylaminoverbindung III,

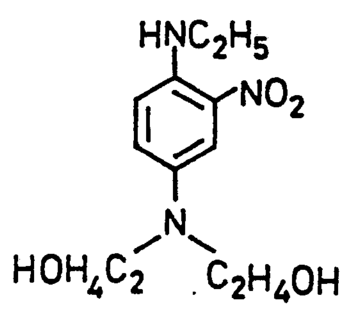

(III)

(vgl. z. B. DE-OS 1 569 808) in Haarfärbemitteln eingesetzt. Die schlechte Löslichkeit des Farbstoffes von unter 0,15 % in Wasser ohne Zusatz von Lösungsvermittlern verhindert jedoch einen Einsatz der Verbindung in Emulsionssystemen in Mengen, die zur Erzielung einer hohen Farbtiefe erforderlich sind.

Im Gegensatz dazu führt die hohe Löslichkeit der in der DE-PS-1 017 750 genannten Trihydroxyethylverbindung IV

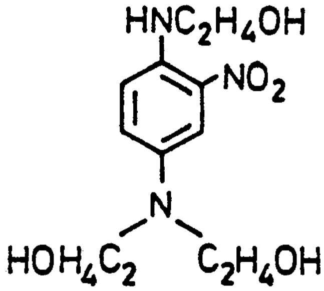

(IV)

zwar zur guten Anwendbarkeit bei hohen Konzentrationen, der Farbstoff wird aber aufgrund der guten Wasserlöslichkeit beim Waschen der Haare sehr schnell wieder ausgespült, so daß die Echtheit nicht zufriedenstellend ist.

Die vorstehend beschriebenen Probleme in anwendungstechnischer Hinsicht treten bei der Verwendung der 1-Methylaminoverbindung V

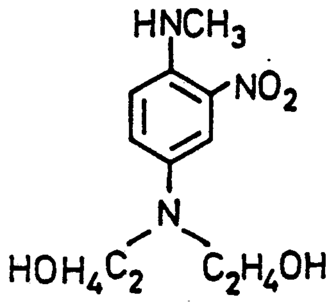

(V)

nach der DE-PS-1 299 002 weniger auf, lediglich die Erzielung dunkler Nuancen kann durch die Löslichkeit von nur etwa 0,5 % erschwert werden. In letzter Zeit sind jedoch Zweifel an der völligen Unschädlichkeit und Ungiftigkeit der Verbindung aufgekommen.

Auch die ebenfalls für Haarfärbemittel empfohlene, zu V isomere Verbindung VI (DE-PS-1 569 807 und CA-PS-900 490)

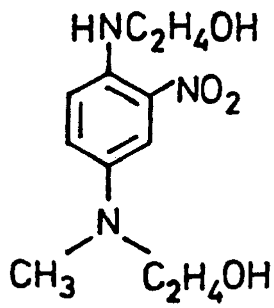

(VI)

kann nicht voll befriedigen. So wird sie einerseits im Amestest auf mutagene Wirkung ungünstig beurteilt, andererseits zeigt sie im Vergleich zu I einen rötlichen Farbstich, der die Nuancierung natürlicher Farbtöne erschwert.

Umso überraschender ist es nach dem zuvor Gesagten, daß der blaue Nitrofarbstoff I, der ein Isomeres zu III und ein Homologes zu VI darstellt, teilweise andere Eigenschaften aufweist, die sich aus den bekannten Verbindungen III bis VI nicht ableiten lassen. So löst sich der erfindungsgemäße Farbstoff I zu etwa 1,0 % in Wasser, was die Erzielung dunkler Nuancen außerordentlich erleichtert, insbesondere im Vergleich zur Verbindung III. Darüberhinaus neigt der Farbstoff I durch seinen niedrigen Schmelzpunkt von 62° C auch beim Überschreiten der Löslichkeit nicht zur Kristallisation, sondern es bildet sich ein Öl, aus dem beim Färbevorgang Farbstoff nachgeliefert werden kann, wohingegen die Wiederauflösung von Kristallen zu lange Zeit benötigt, als daß diese während des Färbeprozesses wieder zur Verfügung stehen könnten. Im Gegensatz zu der ebenfalls gut löslichen Verbindung IV zeigt der erfindungsgemäße Farbstoff jedoch nicht den unerwünschten Nebeneffekt der leichten Auswaschbarkeit.

Die beschriebenen Eigenschaften sind mitverantwortlich für die außerordentliche Lagerstabilität des Farbstoffes I, insbesondere in den nachstehend beschriebenen Färbemitteln. Die Lichtechtheit des Nitrofarbstoffes I ist ebenfalls gut.

Nach dem oben Gesagten spielen für die Auswahl eines Farbstoffes neben den anwendungstechnischen auch die toxikologischen Eigenschaften eine entscheidende Rolle. Überraschenderweise wurde nun gefunden, daß I im Vergleich zu III bis VI als völlig unschädlich angesehen werden kann:

- Der Farbstoff der allgemeinen Formel I ist nicht mutagen in vier Testsystemen:

Ames-Test
Hefezellen-Mutagenitätstest
Mäuse-lymphoma-assay
Human-Lymphozyten-Test

- Der Farbstoff weist eine hohe $LD_{50}$ im Bereich 2 g/kg Körpergewicht auf.
- Der Farbstoff zeigt eine gute Hautverträglichkeit.

Die Herstellung des erfindungsgemäßen Farbstoffes kann auf zwei Wegen erfolgen, nämlich entweder durch Ethylierung des 1,4-Bis-(2'-hydroxyethyl-amino)-2-nitrobenzols oder durch Ethylierung des 2-Nitro-p-phenylendiamins und anschließende Hydroxyethylierung an N-1 und N-4. Die Herstellung auf dem zweiten weg wird im Herstellungsbeispiel beschrieben.

Gegenstand der vorliegenden Anmeldung sind daher Mittel zur Färbung von Haaren mit einem Farbstoffgehalt und üblichen kosmetischen Zusätzen, dadurch gekennzeichnet, daß sie die Verbindung der Formel I enthalten. Die erfindungsgemäßen Mittel zur Färbung von Haaren betreffen insbesondere solche, die ohne Zugabe eines Oxidationsmittels angewendet werden. Solche Haarfärbemittel enthalten neben dem erfindungsgemäßen blauen Nitrofarbstoff der angegebenen Formel I noch andere direkt auf das Haar aufziehende Farbstoffe. Von diesen für die Haarfärbung bekannten Farbstoffen seien beispielsweise die folgenden Klassen erwähnt:

Aromatische Nitrofarbstoffe (z. B. 1,2-Diamino-4-nitrobenzol) 1-Amino-4-(2',3'-dihydroxypropyl)-amino-5-chlor-2-nitro-benzol, 1-Amino-2-(2'-hydroxyethyl)-amino-5-nitro-benzol, 1-Amino-2-nitro-4-bis-(2'-hydroxyethyl)-amino-benzol, 4-(2'-Ureido-ethyl)-amino-nitrobenzol, Pikraminsäure, 2-(2'-Hydroxyethyl)-amino-4,6-dinitro-phenol, 4-(2',3'-Dihydroxypropyl)-amino-3-nitro-toluol, 4-(2'-Hydroxyethyl)-amino-3-nitro-chlorbenzol, Azofarbstoffe (z. B. Acid Brown 4, C. I. 805), Anthrachinonfarbstoffe (z. B. Disperse Blue 23, C. I. 61 545 und Disperse Violet 4, C. I. 61 105), Triphenylmethanfarbstoffe (z. B. Basic Violet 1, C. I. 61 100), wobei die Farbstoffe dieser Klassen je nach der Art ihrer Substituenten sauren, nichtionogenen oder basischen Charakter haben können. Weitere geeignete direkt auf das Haar aufziehende Farbstoffe sind beispielsweise in dem Buch von J. C. Johnson, "Hair Dyes" Noyes Data Corp. Park-Ridge (USA) 1973) beschrieben.

Mit Haarfärbemitteln, die derartige Gemische von Farbstoffen enthalten, lassen sich Naturtöne, modische Blond- und Brauntöne und leuchtende Modetöne von hervorragender Stabilität herstellen.

Die Zubereitungsform der hier beschriebenen Haarfärbemittel auf der Basis von direkt auf das Haar aufziehenden Farbstoffen kann beispielsweise eine Lösung, insbesondere eine wäßrige oder wäßrigalkoholische Lösung sein. Bevorzugte Zubereitungsformen sind weiterhin eine Creme, ein Gel oder eine Emulsion, wobei sie auch im Gemisch mit einem Treibgas oder mittels einer Pumpe versprüht werden können.

Übliche Zusätze in Lösungen, Cremes, Emulsionen oder Gelen sind zum Beispiel Lösungsmittel wie Wasser, niedere aliphatische Alkohole, beispielsweise Ethanol, Propanol, Isopropanol, Glycerin oder Glykole wie Ethylenglykol und Propylenglykol, oder auch Glykolether, weiterhin Netzmittel oder Emulgatoren aus den Klassen der anionischen, kationischen, amphoteren oder nichtionogenen oberflächenaktiven Substanzen wie Fettalkoholsulfate, Fettalkoholethersulfate, Alkylsulfonate, Alkylbenzolsulfate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Bentonit, Stärke, Polyacrylsäure, Cellulosederivate, wie Carboxymethylcellulose, Alginate, Vaseline, Paraffinöl und Fettsäuren sowie außerdem Pflegestoffe wie Lanolinderivate, Cholesterin, Pantothensäure und Betain, weiterhin Parfümöle und Komplexbildner. Die erwähnten Bestandteile werden in den für solche Zwecke üblichen Mengen verwendet, zum Beispiel die Netzmittel und Emulgatoren in Konzentrationen von etwa 0,5 bis 30 Gew.-%, während die aus diesen beiden Säuren und Aminoalkoholen beziehungsweise deren Salze oder Quaternisierungsnprodukte, Polyacrylnitril, Polyvinyllactame sowie Copolymerisate aus derartigen Verbindungen wie Polyvinylpyrrolidon-Vinylacetat und dergleichen erwähnt.

Auch natürliche Polymere, wie Chitosan (entacetyliertes Chitin) oder Chitosanderivate, können für den genannten Zweck eingesetzt werden.

Die Polymerisate sind in diesen Mitteln in der üblichen Menge von etwa 1 bis 4 Gew.-% enthalten. Die pH-Werte der Mittel liegen im Bereich von etwa 5,0 bis 9,5.

Die Anwendung dieser Haarfärbemittel mit zusätzlicher Festigung erfolgt in bekannter und üblicher Weise durch Befeuchten des Haares mit dem Festiger, Festlegen (Einlegen) des Haares zur Frisur und anschließende Trocknung.

Selbstverständlich können die vorstehend beschriebenen Haarfärbemittel gegebenenfalls weitere gebräuchliche kosmetische Zusätze wie z. B. Pflegestoffe, Netzmittel, Verdicker, Welchmacher, Konservierungsstoffe, Antischuppenwirkstoffe und Parfümöle enthalten.

Die nachstehenden Beispiele sollen den Anmeldungsgegenstand erläutern, ohne ihn darauf zu beschränken.

Verdicker in einer Menge von etwa 0,1 bis 25 Gew.-% in den Zubereitungen entnalten sein können.

Der Farbstoff der Formel I soll in diesen Haarfärbemitteln in einer Konzentration von etwa 0,01 bis 2,0 Gew.-% vorzugsweise von 0,01 bis 1,0 Gew.-%, enthalten sein. Der Gesamtgehalt an Farbstoffen liegt in den Grenzen von etwa 0.01 bis 3,0 Gew.-%.

Der pH-Wert dieser Färbemittel liegt im Bereich von 3 bis 10,5, insbesondere bei pH 7,5 bis 9,5, wobei die Einstellung des gewünschten pH-Wertes hauptsächlich mit Ammoniak erfolgt, jedoch auch mit organischen Aminen wie beispielsweise Monoethanolamin oder Triethanolamin vorgenommen werden kann.

Ihre Anwendung erfolgt in üblicher Weise durch Aufbringen des Mittels auf die Haare, mit denen es eine Zeitlang, zwischen 5 und 30 Minuten, in Berührung bleibt. Anschließend wird mit Wasser, gegebenenfalls noch mit einer schwachen organischen Säure, gespült und getrocknet. Als schwache organische Säure können beispielsweise Essigsäure, Zitronensäure, Weinsäure und ännliche Verwendung finden.

Die vorstehend beschriebenen Haarfärbemittel können selbstverständlich auch kosmetische Polymerisate enthalten, wodurch gleichzeitig mit der Färbung eine Festigung der Haare erreicht wird. Solche Mittel werden im allgemeinen als Tönungsfestiger oder Farbfestiger bezeichnet.

Von den für diesen Zweck in der Kosmetik bekannten Polymerisaten seien beispielsweise Polyvinylpyrrolidon, Polyvinylacetat, Polyvinylalkohol oder Polyacrylverbindungen wie Acrylsäurebeziehungsweise Methacrylsäurepolymerisate, basische Polymerisate von Estern.

## Haarfärbebeispiele

### Beispiel 1

Das flüssige Haarfärbemittel der Zusammensetzung

| | |
|---|---|
| 1,00 g | Farbstoff nach Formel I |
| 0,50 g | Hydroxyethylcellulose |
| 5,00 g | Laurylalkohol-diglykolethersulfat-Natriumsalz, 28 %-ige wäßrige Lösung |
| 15,00 g | Isopropylalkohol |
| 0,03 g | Ammoniak, 25 %-ig |
| 78,47 g | Wasser |
| 100,00 g | |

wird auf weiße menschliche Haare aufgetragen und 10 Minuten einwirken gelassen. Nach Spülung mit Wasser und Trocknung ist das Haar dunkelblau gefärbt.

### Beispiel 2

Farbfestiger

| | |
|---|---|
| 0,02 g | Farbstoff nach Formel I |
| 2,00 g | Polyvinylpyrrolidon |
| 0,10 g | Glycerin |
| 40,00 g | Isopropylalkohol |
| 57,88 g | Wasser |
| 100,00 g | |

Weiße menschliche Haare werden mit der festigenden Färbelösung zur Frisur eingelegt und getrocknet. Dem Haar wird ein angenehmer silber-blauer Schimmer verliehen.

**Beispiel 3**

Haarfärbemittel in Gelform

| | |
|---|---|
| 1,0 g | Farbstoff nach Formel I |
| 0,3 g | 1-Amino-4-(2',3'-dihydroxypropyl)-amino-5-chlor-2-nitrobenzol |
| 0,1 g | 4-(2'-Ureidoethyl)-amino-nitrobenzol |
| 17,5 g | Ölsäure |
| 7,5 g | Isopropanol |
| 9,0 g | Ammoniak 25 %-ig |
| 64,6 g | Wasser |
| 100,00 g | |

Das vorstehende Haarfärbemittel wird auf graue menschliche Haare aufgetragen und 30 Minuten einwirken gelassen. Sodann wird mit Wasser gespült und getrocknet. Das Haar hat eine dunkelblonde Färbung mit mahagonifarbenen Reflexen erhalten.

**Beispiel 4**

Haarfärbemittel in Cremeform

| | |
|---|---|
| 0,3 g | Farbstoff nach Formel I, als Hydrochlorid eingesetzt |
| 0,05 g | 1-Amino-2-(2'-hydroxyethyl)-amino-5-nitro-benzol |
| 0,32 g | 1-Amino-2-nitro-4-bis-(2'-hydroxyethyl)-aminobenzol |
| 0,025 g | Disperse Blue 23, C. I. 61 545 |
| 7,5 g | Cetylalkohol |
| 1,75 g | Laurylalkohol -diglykolethersulfat-Natriumsalz 28 %-ige wäßrige Lösung |
| 0,1 g | p-Hydroxybenzoesäuremethylester |
| 0,2 g | Ammoniak, 25 %-ig |
| 90,055 g | Wasser |
| 100,00 g | |

50 g des vorstehenden Haarfärbemittels werden auf weiße menschliche Haar aufgebracht und nach einer Einwirkungszeit von 20 Minuten mit Wasser ausgespült. Das Haar wird anschließend getrocknet. Es ist in einem natürlichen braunen Farbton gefärbt.

**Herstellungsbeispiel**

1. Stufe

1-Amino-4-benzolsulfonamido-2-nitro-benzol

153 g (1,0 Mol) 1,4-Diamino-2-nitro-benzol werden in 525 ml 24 %-iger Kaliumhydroxidlösung (2,2 Mol) aufgeschlämmt. Unter Rühren läßt man 193 g (1,1 Mol) Benzolsulfonsäurechlorid zutropfen. Es wird kurze Zeit erwärmt, wobei ein gelber Niederschlag ausfällt. Der Ansatz wird auf Wasser gegossen und vom Niederschlag unter Nachwaschen mit Wasser abfiltriert. Der Rückstand wird getrocknet, man erhält das Sulfonamid in nahezu quantitativer Ausbeute. Der Schmelzpunkt nach Umkristallisation aus Dioxan beträgt 162 - 163° C.

2.Stufe

1-Amino-4-N-ethyl-benzolsulfonamido-2-nitro-benzol

58,6 g (0,2 Mol) des Sulfonamids aus Stufe 1 werden in 300 ml Ethanol gelöst und mit einer Lösung von 17 g (0,3 Mol) Kaliumhydroxid in wenig Wasser und mit 31 g (0,2 Mol) Jodethan versetzt. Man erwärmt auf 70° C und setzt nach 5 Stunden noch einmal 5,6 g (0,1 Mol) Kaliumhydroxid in wenig Wasser und 15,2 g (0,1 Mol) Jodethan zu. Nach weiteren 3 h läßt man abkühlen, wobei die Ethylverbindung in Form gelber Blättchen auskristallisiert. Man erhält 25 g (39 % der Theorie) an Ethylverbindung vom Schmelzpunkt 152° C.

3.Stufe
1-Amino-4-ethylamino-2-nitro-benzol

25,0 g (87 mMol) des Sulfonamids aus Stufe 2 werden in 50 ml 50 %-iger (Volumen-Prozent) Schwefelsäure 30 Minuten auf 130°C erwärmt. Nach dieser Zeit tritt beim Verdünnen einer Probe mit Wasser keine Trübung mehr auf, damit ist das Sulfonamid vollständig gespalten. Man gießt auf Wasser und neutralisiert mit Ammoniak. Beim Erkalten erstarrt die sich zunächst ölig abscheidende Base.

Man erhält 13,0 g (93 % der Theorie) an 4-Ethylaminover-bindung vom Schmelzpunkt 53°C.

4. Stufe
4-(N-Ethyl,N-2'-hydroxyethyl)-amino-1-(2''-hydroxyethyl)-amino-2-nitro-benzol

13 g (72 mMol) 1-Amino-4-ethylamino-2-nitro-benzol aus Stufe 3 werden in 23 g (286 mMol) 2-Chlorethanol gelöst. Es wird auf 140°C erwärmt und unter Rühren langsam eine Lösung von 11,5 g (286 mMol) Natriumhydroxid in 110 ml Wasser zugetropft. Nach 5 h wird noch einmal die gleiche Menge 2-Chlorethanol zugesetzt und wiederum die entsprechende Menge Natronlauge zugetropft. Nach 10 h ist die Alkylierung weitgehend beendet, man läßt abkühlen und extrahiert mit Essigsäurethylester Die vereinigten Essigesterphasen werden über Natriumsulfat getrocknet und das Lösungsmittel im Vakuum verdampft. Der ölige Rückstand wird in 30 ml Isopropanol gelöst und mit 5 ml konzentrierter Salzsäure versetzt. Das Hydrochlorid fällt in Form gelber Kristalle aus, es wird abfiltriert und der Rückstand getrocknet. Man erhält 6 g (27 % der Theorie) des Hydrochlorids von I, das mit wenig 1-Amino-4-(N-ethyl,N-2'-hydroxyethyl)-amino-2-nitro-benzol verunreinigt ist.

Der reine Farbstoff I läßt sich aus dem öligen Rückstand der Essigesterextraktion gewinnen durch Chromatographie an Kieselgel mit Essigester als Laufmittel. Die reine Base schmilzt bei 62°C.

Elementaranalyse:

| | berechnet | gefunden |
|---|---|---|
| C | 53,52 | 53,72 |
| H | 7,11 | 7,17 |
| N | 15,60 | 15,54 |

Alle in der Anmeldung angegebenen Prozentzahlen sollen Gewichtsprozente darstellen.

## Patentansprüche

1. 4-(N-Ethyl, N-2'-hydroxyethyl-amino-1-(2''-hydroxyethyl)-amino-2-nitro-benzol der Formel I

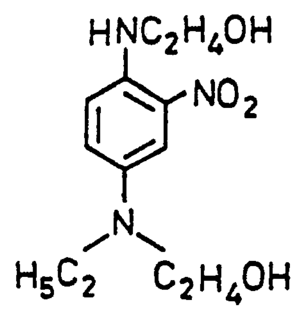

(I)

2. Mittel zur Färbung von Haaren mit einem Farbstoffgehalt und üblichen kosmetischen Zusätzen, dadurch gekennzeichnet, daß es die Verbindung nach Anspruch I enthält.

3. Mittel nach Anspruch 2, dadurch gekennzeichnet, daß es 0,01 bis 2,0 Gew.-% des Farbstoffes nach Anspruch 1 enthält.

4. Mittel nach Anspruch 2 und 3, zusätzlich enthaltend bekannte, direkt auf das Haar aufziehende Farbstoffe, die ausgewählt sind aus 1-Amino-4-(2',3'-dihydroxypropyl)-amino-5-chlor-2-nitro-benzol, 4-(2'-Ureidoethyl)-amino-nitrobenzol, 1-Amino-2-(2'-hydroxyethyl)-amino-5-nitro-benzol, 1-Amino-2-nitro-4-bis-(2'-hydroxy-ethyl)-amino-benzol, Pikraminsäure) 2-(2'-Hydroxyethyl)-amino-4,6-dinitro-phenol, 4-(2',3'-Dihydroxy-propyl)-amino-3-nitro-toluol, 4-(2'-Hydroxyethyl)-amino-3-nitro-chlorbenzol, Acid Brown 4 (C I. 14 805), Disperse Violet 4(C. I. 61 105), Disperse Blue 23 (C I. 61 545) und Basic Violet 1 (C. I. 61 100).

5. Mittel nach Anspruch 2 bis 4, zwecks Haarfestigung enthaltend in wäßrig-alkoholischer Lösung ein kosmetisches Polymerisat.

6. Mittel nach Anspruch 2 bis 5, dadurch gekennzeichnet, daß es einen pH-Wert von 6,0 bis 9,5 aufweist.

**Claims**

1. 4-(N-ethyl, N-2'-hydroxyethyl)-amino-1-(2"-hydroxyethyl)-amino-2-nitrobenzene of formula I

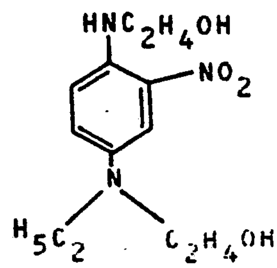

(I)

2. Composition for dying hair with a dye content and usual cosmetic additives, characterised in that it contains the compound according to claim 1.

3. Composition according to claim 2, characterised in that it contains 0.01 to 2.0 % by weight of the dye according to claim 1.

4. Composition accordinG to claims 2 and 3 containing in addition known dyes, which can be absorbed directly onto the hair and which are selected from 1-amino-4-(2',3'-dihydroxypropyl)-amino-5-chloro-2-nitrobenzene, 4-(2'-ureidoethyl)-amino-nitrobenzene, 1-amino-2-(2'-hydroxyethyl)-amino-5-nitrobenzene, 1-amino-2-nitro-4-bis-(2'-hydroxyethyl)-amino-benzene, picramic acid, 2-(2'-hydroxyethyl)-amino-4,6-dinitrophenol, 4-(2',3'-dihydroxypropyl)-amino-3-nitrotoluene, 4-(2'-hydroxyethyl)-amino-3-nitro-chlorobenzene, Acid Brown 4 (C. I. 14 805), Disperse Violet 4 (C. I. 61 105), Disperse Blue 23 (C. I. 61 545) and Basic Violet I (C. I. 61 100).

5. Composition according to claims 2 to 4, for use as a setting lotion and containing a cosmetic polymer in an aqueous-alcoholic solution.

6. Composition according to claim 2 to 5, characterised in that it has a pH-value of 6.0 to 9.5.

**Revendications**

1. 4-(N-éthyl, N-2'-hydroxyéthyl)-amino-1-(2"-hydroxy-éthyl)-amino-2-nitrobenzène répondant à la formule I

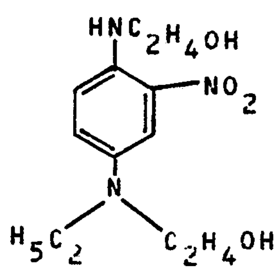

(I)

2. Produit de coloration des cheveux renfermant un colorant et des additifs cosmétiques usuels, caractérisé en ce qu'il renferme le composé selon la revendication 1.

3. Produits selon ta revendication 2, caractérisé en ce qu'il renferme 0,01 à 2.0 % en poids du colorant selon la revendication 1.

4. Produits selon les revendications 2 et 3, renfermant en outre des colorants connus prenant directement sur les cheveux, qui sont choisis parmi le 1-amino-4-(2', 3'-dihydroxypropyl)-amino-5-chloro-2-nitrobenzène, le 4-(2'-uréidoéthyl)-amino-nitro-ben-zène, le 1-amino-2-(2'-hydroxyéthyl)-amino-5-nitro-benzène, le 1-amino-2-nitro-4-bis-(2'-hydroxy-éthyl)-amino-benzène, l'acide picramique, le 2-(2'-hydroxyéthyl)-amino-4,6-dinitrophénol, le 4-(2',3'-dihydroxypropyl)-amino-3-nitrotoluène, le 4-(2'-hydroxyéthyl)-amino-3-nitro-chlorobenzène, Acid Brown 4 (I. C. 14 805), Disperse Violet 4 (I. C. 61 105), Disperse Blue 23 (I. G. 61 545), et Basic Violet 1 (I. G. 61 100).

5. Produit selon les revendications 2 à 4, renfermant aux fins des fixation des cheveux, en solution hydroalcoolique, un produit de polymérisation cosmétique.

6. Produit selon les revendications 2 à 5, caractérisé en ce qu'il présente une valeur de pH de 6,0 à 9,5.